Europäisches Patentamt

European Patent Office

Office européen des brevets

(11) Publication number: **0 192 453**
**A2**

# (12) EUROPEAN PATENT APPLICATION

(21) Application number: 86301101.1

(51) Int. Cl.⁴: **A 61 M 5/32**

(22) Date of filing: 18.02.86

(30) Priority: 18.02.85 NZ 211150

(43) Date of publication of application:
27.08.86 Bulletin 86/35

(84) Designated Contracting States:
AT BE CH DE FR GB IT LI LU NL SE

(71) Applicant: Lixater Holdings Limited
16 Liverpool Street
Auckland(NZ)

(72) Inventor: Law, Ronald
Road 1
Henderson Auckland(NZ)

(72) Inventor: Goldwater, Paul Nathan
31 St. Georges Bay Road
Parnell Auckland(NZ)

(72) Inventor: Nixon, Albert Dunsmuir
98 Bramley Drive
Pakuranga Auckland(NZ)

(74) Representative: Cardwell, Stuart Martin et al,
Roystons Tower Building Water Street
Liverpool, Merseyside L3 1BA(GB)

(54) Needle guard.

(57) A needle guard (10) has a centrally disposed aperture (14, 41, 51) for the reception of a tubular needle cover (22) used to house disposable needles for hypodermic syringes.

The needle guard (10) is formed from a needle puncture resistant but resilient plastic so that it can frictionally grip the needle cover (22) as the guard is slid over the cover. The cover is cup shaped with outwardly extending flanges (16) to prevent it from rolling when placed on a surface. The cover can be ejected from the guard and its use with a disposable container (30) is described.

EP 0 192 453 A2

Croydon Printing Company Ltd.

Title:   Needle Guard

DESCRIPTION

This invention relates to a guard for needles or like instruments.

Needles used on hypodermic syringes or those used to take blood samples are typically provided with a needle cover in the form of a tubular sleeve having a closed end so that the tip of the needle is encased within the sleeve.   Soiled or used needles can give rise to the risk of transmission of diseases such as Hepatitus B or AIDS as it is not uncommon for health professionals to prick themselves with needles after use as they endeavour to dispose of the needles or insert them into their needle covers prior to disposal.   This problem is so serious that the Centre for Disease Control recommends that the used needles are not returned to their needle covers.

Various needle packages have been proposed but none of them have solved the problem of how to avoid the risk of needle pricks with the conventional tubular needle covers which are widely used today.

Examples of such prior art needle packages include:

U. S. Patent 4,287,988 - Syringe sheath guide

U. S. Patent 3,101,841 - Needle package

U. S. Patent 3,149,717 - Container for hypodermic needle

U. S. Patent 3,294,331 - Dental needle shield

U. S. Patent 3,329,146 - Needle container

U. S. Patent 3,333,682 - Disposable needle container

U. S. Patent 3,342,319 - Rigid tubular syringe package

It is an object of this invention to provide a needle guard which can be used with tubular needle covers and which minimises the risk of needle pricks when inserting a needle into its needle cover, or removing it therefrom.

In one aspect, the invention provides a needle guard for use with a tubular cover having an opening at one end for a disposable hypodermic needle or the like, said guard including:

a body of needle puncture resistant material having an aperture therethrough capable of receiving and holding the cover as it is positioned in the aperture, said body extending outwardly from the aperture so as to shield a user's hand from a needle as it is inserted into the cover held in the body.

Preferably the body is in the form of a cup or socket having an aperture therein capable of receiving a needle cover. Alternatively, the body may be in the form of an apertured disc or other shape to form a shield or guard about the mouth of the needle cover.

In a second aspect, the invention provides a method of handling needles or like instruments wherein a needle within a needle cover is provided with an outwardly extending shield portion, the needle is removed from its cover and after use is inserted into a shielded needle cover which is held with the shield between the user's hand and the entrance end of the cover. Optionally the used needle and its needle cover may be ejected from the shield portion.

In a third aspect, the invention provides a container for used needles having an aperture therein capable of allowing a needle cover to be pushed therethrough but small enough to prevent the entry of a needle guard as described in any of the previous aspects of this invention.

These and other aspects of this invention, which should be considered in all its novel aspects, will become apparent from the following description, which is given by way of example only, with reference to the
accompanying drawings, in which:

Figure 1 is a cross section through a preferred needle
guard;

Figure 2 shows a cut-away view of the needle guard,
with a needle cover in place, showing how it can be held
in the hand whilst a needle is in a position to be inserted
into its cover;

Figure 3 shows a container for the disposal of
needles and needle covers, with a needle guard positioned
on the container;

Figure 4 is a side view of the needle guard of
Figure 1;

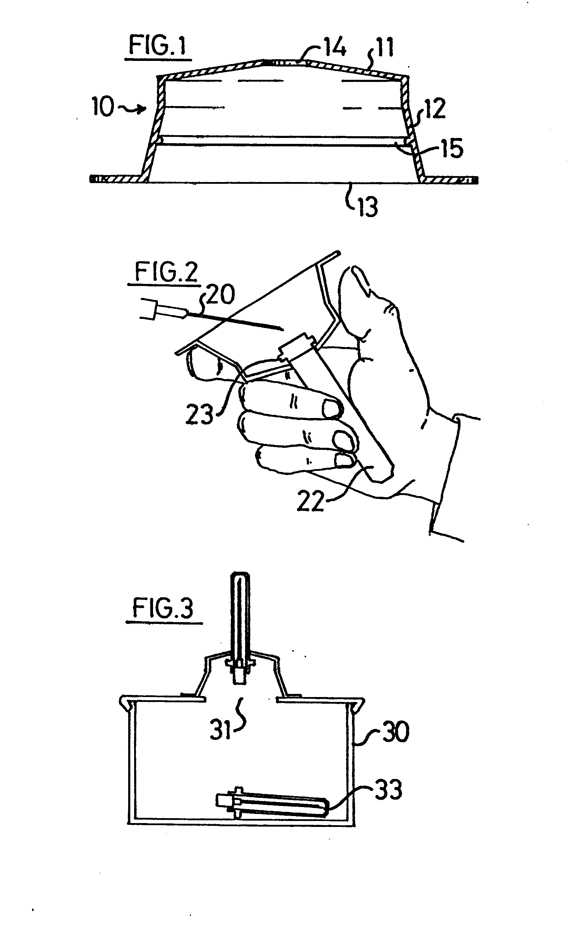

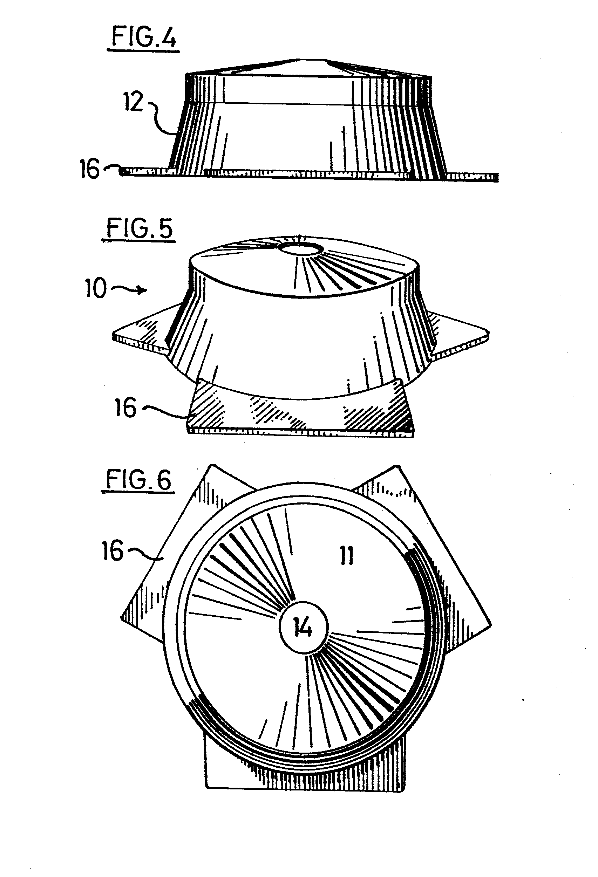

Figure 5 is a perspective view of the needle guard
of Figure 1;

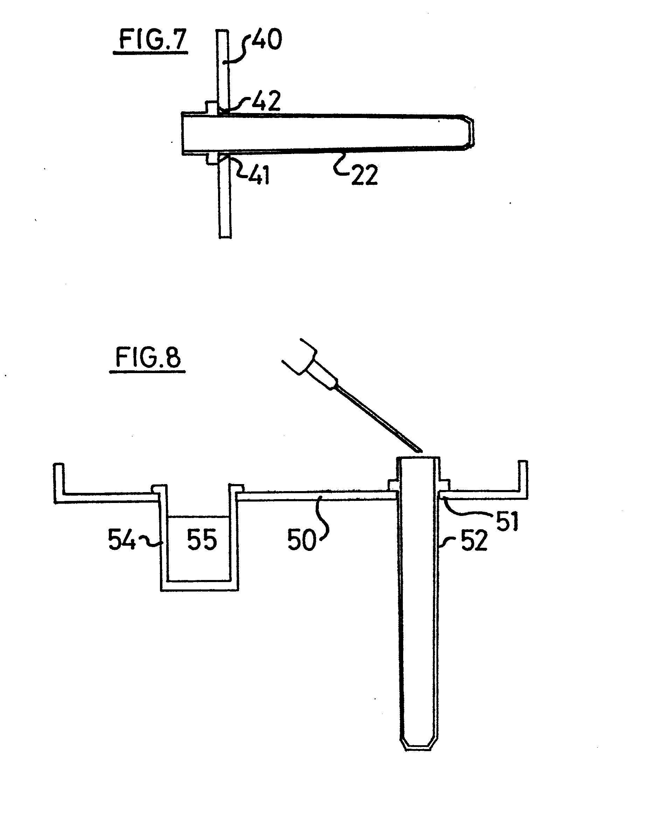

Figure 6 is a top plan view of the needle guard of
Figure 1;

Figure 7 shows a cross-section through an
alternative needle guard, positional on a needle cover;

Figure 8 shows a cross-section through a further
needle guard, having provision for a needle cover and
container for a liquid.

Turning now to Figures 1 to 6, a preferred needle
guard 10 is in a form of a cup or socket which is
conveniently formed from a plastics material, although
other materials may be used.　The material is preferably
resilient enough to assist in gripping a needle cover,
but is tough enough to prevent a needle being inadvertently
pushed therethrough.

The needle guard 10 is formed with a base 11 and
sidewalls 12.

As shown, the sidewalls 12 may be slightly tapered to
present a relatively wide entry 13 with the sidewalls 12
assisting in deflecting or directing a needle towards the approximately central aperture 14.

Optionally, an internal rib 15 may be provided of such a size that the needle guard can be clicked over the end of a needle holder (not shown), so that the guard can be conveniently held in place when not required for use with a cover.

Although the needle guard may be made of any convenient size, we have found that a suitable size for the needle guard involves a base of diameter of approximately 32 millimetres, and an entrance 13 of approximately 38 millimetres.   This enables a single guard size to be produced from one mould but allowing for aperture sizes 14 (created by using different mould pins) to thus accommodate different sizes of needle covers from small to large.

Although these sizes are given by way of example only and are not to be construed as in any way limiting the invention, it will be appreciated that such a size enables the guard 10 to provide a reasonable shield for the user's hand (see Figure 2) whilst inserting a needle into a needle cover, yet on the other hand the needle guard is of relatively compact size and thus may be readily carried by a user and/or packed with a supply of needles, and/or attached to the end of a needle holder e.g. the type of holder for use with needles for the collection of blood samples).

Preferably, the needle guard 10 is provided with anti-roll means on the exterior thereof to minimise risk of the needle guard rolling off a bench or the like. This is conveniently achieved by providing flats 16 on the exterior of the sidewall 12, preferably three such flats as shown in Figure 6.   Alternatively, the needle guard can be provided with a plurality of sidewalls, e.g. the needle guard may be square, hexagonal, or any other polygonal shape when viewed in plan.

Figure 2 also shows a hypodermic needle 20 which is push-fit within a needle cover 22. This cover 22 typically has a shoulder 23 adjacent its open end.

Figure 2 shows a needle cover 22 inserted through the aperture 14 of a needle guard 10 so that its shoulder 23 abuts against the underside of the base 11. In this position, the needle cover 22 may be held in one hand and the needle pulled out of the needle cover (typically when the needle is attached to a syringe or needle holder). After use, the needle can then be inserted into the needle cover with minimal risk of the user being pricked by the needle, as the user's hand is shielded by the needle guard as the needle is presented towards the open end of the needle cover. When the needle is inserted into the needle cover it may be disengaged from the syringe or other needle holder. The spent needle (and needle cover) can then be removed by pushing it out of the needle guard.

Preferably a disposal container 30 is provided having an aperture 31 in the lid thereof just slightly larger than the diameter of the needle cover and its associated shoulder 23, but with the aperture 31 in the lid being less than the diameter of the needle guard. By this means, the needle guard can be placed over the aperture 31 and the needle cover pushed downwardly in the direction of arrow 32 so that it is ejected directly into the container as shown by spent needle and needle cover 33.

In order to facilitate the gripping action of the needle guard about the needle cover, it is preferred that the aperture 14 is tapered so that it presents a slightly larger diameter on the inner side of the base 11 than on the outer side of the base 11. This also facilitates the insertion and/or ejection of the needle cover from

- 6 -                                               0192453

aperture 14.

Figures 7 and 8 show alternative embodiments. Figure 7 shows a needle guard in the form of a flat disc or washer 40 which grips the cover 22 and functions in a similar fashion to the guard of Figure 1. It can be a round disc, or more preferably rectangular or other non-round shape to prevent the guard and cover from rolling when placed on a surface. The washer 40 preferably has a central aperture 41 with a taper 42 to facilitate gripping of the needle cover 22 as it is slid over the cover.

The needle guard 50 of Figure 8 has an off-centre aperture 51 for a needle cover 52 and one or more chambers 54 for liquids 55. Chamber 54 is conveniently formed by a container which fits into an aperture 55 in a similar fashion to the needle cover 52 fitting into aperture 51. By this means, the guard 50 can be used for blood tests or the like, as well as a suitable holder for the needle cover 52. Such a needle guard can be larger than that of Figure 1, and may require a separate stand to support it in the upright position when not held in the hand.

Finally, it will be appreciated that various alterations or modifications may be made to the foregoing without departing from the scope of this invention as exemplified by the following claims.

## CLAIMS

1. A needle guard (10) for use with a tubular cover (22) having an opening at one end for a disposable hypodermic needle or the like, characterised in that said guard comprises a body (10, 40, 50) of needle puncture resistant material having an aperture (14, 41, 51) therethrough capable of receiving and holding the cover (22) as it is positioned in the aperture, said body extending outwardly from the aperture so as to shield a user's hand from a needle as it is inserted into the cover held in the body.

2. A needle guard as claimed in claim 1, wherein the body (10, 40, 50) is formed from a resilient plastics material.

3. A needle guard as claimed in claim 1 or 2, wherein the body is in the form of an annular disc (40).

4. A needle guard as claimed in claim 1 or 2, wherein the body is in the form of a cup or socket having an apertured end wall (11) surrounded by a side wall or walls (12).

5. A needle guard as claimed in any preceding claim, wherein the body has anti-roll means about its periphery.

6. A needle guard as claimed in claim 5, wherein said anti-roll means consists of outwardly extending flanges (16).

7. A needle guard as claimed in any one of the preceding claims, in combination with a container (30) for disposal of spent needles and covers (22), said container having an aperture (31) therein larger than the cover diameter but smaller than the body diameter to allow the body to be positioned on the container about the container aperture with the cover protruding away from the container so that the cover can be pushed through the body and into the container.

0192453

8. A needle guard as claimed in any one of claims 1 to 6, and further including one or more chambers (54) attached to the body (50) and spaced apart from said aperture (51).

9. A needle guard as claimed in claim 8, wherein said one or more chambers (54) includes a receptacle for a liquid which can be held in the hand thereby allowing a needle to be inserted into the liquid or into the needle cover (22).

FIG.1

14  11

10 →

12

15

13

FIG.2

20

23

22

FIG.3

31

30

33

FIG.4

12

16

FIG.5

10

16

FIG.6

16

11

14

FIG.7

40

42

41

22

FIG.8

54

55

50

52

51